# EUROPEAN PATENT APPLICATION

(11) **EP 4 005 565 A1**
(43) Date of publication of application: **01.06.2022**
(21) Application number: 20210604.3
(22) Date of filing: 30.11.2020
(51) Int. Cl.: A61K 31/13, A61P 31/16

(54) **NOVEL USES OF ADAMANTANE DERIVATIVES**

(71) Applicant: Merz Pharmaceuticals GmbH, 60318 Frankfurt am Main (DE)
(72) Inventor: FINK, Klaus, 60318 Frankfurt/Main (DE)
(74) Representative: Graf Keyserlingk, Nikolai

(57) **Abstract**

Adamantane derivative selected from the group of amantadine or memantine as free base or in the form of its pharmaceutically acceptable salt for use in the treatment of a patient suffering from an infection with SARS-CoV-2 wherein said amantadine derivative is administered topically to the respiratory system of said patient.

## Description

### FIELD OF THE INVENTION

This invention relates to adamantane derivatives selected from the group of amantadine or memantine as free base or in the form of their pharmaceutically acceptable salts for use in the treatment of a patient suffering from an infection with SARS-CoV-2 wherein said adamantane derivatives are administered topically to the respiratory system of said patient.

### BACKGROUND OF THE INVENTION

Since the SARS-CoV-2 pandemic started in December 2019 the search for protective vaccines and for drug treatments has become mandatory to fight the health and economic consequences. Although travel restrictions, social distancing and face masks are suitable counter measures they turned out not to bring the pandemic under control. Vaccines and antiviral drugs are urgently needed. Even if vaccines will be available the need for antiviral agents against SARS-CoV-2 will persist because vaccinating a major part of the population takes a long time, the required vaccine may not be available in due time, the population in less wealthy countries may be not or incompletely immunized. It is even conceivable that patients may suffer from COVID-19 disease although they were vaccinated. These limitations will result in continuing SARS-CoV-2 infections or local outbreaks that can be treated with inexpensive and broadly available antiviral drugs. However, for none of the repurposed antiviral drugs, that would fulfil these requirements, unequivocal evidence for clinical efficacy against SARS-CoV-2 after systemic administration has been demonstrated so far.

Adamantanes are organic compounds having three cyclohexane rings fused to each other. The molecule is both rigid and virtually stress-free. Adamantane is the most stable isomer of C₁₀H₁₆. Adamantane derivatives are well known as pharmaceutically active compounds with broad use in different therapeutic fields. In particular known for their pharmaceutical properties and widely used are amantadine and memantine.

Amantadine has been shown effective against influenza A infections in the 1960s and was approved in 1966 in Germany for the prophylaxis of Asian influenza and in 1976 by the FDA for the treatment of influenza A. Amantadine is also approved for the treatment of Parkinson's disease. Amantadine has been used in many countries for influenza A treatment until point mutations in the influenza A genome resulted in increasing drug resistance and the US Center for Disease Control CDC recommended in 2009 not to use it for influenza A anymore. The 2008/2009 seasonal influenza (H3N2) and the 2009 swine flu pandemic (H1N1) were almost completely resistant to amantadine (CDC, 2009). Recently the efficacy of amantadine against SARS-CoV-2 has been demonstrated in vitro (Rodon J, 2020; Fink et al., 2020 in prep).

However, the concentrations of amantadine and memantine in the cell culture medium required for replication inhibition by 50% (IC50) in SARS-CoV-2 infected Vero E6 cells ranged between 20 and 105 µM. Assuming that the concentration in cell culture medium in vitro corresponds to the drug plasma concentration in a patient in vivo, the IC50 for systemically administered amantadine has to be considered higher than achievable by the approved dosing regimens and exceeds substantially the serum concentration range without unintended toxic symptoms. Toxic symptoms, mostly psychotic symptoms, have been observed at plasma concentrations exceeding 6.6 µM; hallucinations were experienced between 14.9 and 29.1 µM, depression and confusion were observed at 19.9 µM, aggressive behaviour at 5.5-6.6 µM and seizures at 13.2 µM. Albrektson (Albrektson et al., 2018) reported an amantadine intoxication case with encephalopathy, agitation, respiration problems, hypoxia, but no renal functional impairment after intake of 1000 mg/day resulting in serum concentrations of 15.9 µM. In patients receiving regularly 600 mg amantadine orally per day plasma concentrations of up to 14.6 µM were found (Brenner et al., 1989). Higher doses have not been used for any indication. Thus, the concentration required for inhibition of SARS-CoV-2 replication in Vero E6 cells is 5-8 times higher than the plasma concentrations observed in patients after therapeutic doses (200-600 mg/day peroral) of amantadine which indicates that systemically administered amantadine does not offer a therapeutic window between antiviral efficacy in vitro and cytotoxicity in vitro or systemic toxicity in vivo. As a result, oral or i.v. administration of high amantadine doses for COVID-19 treatment appears to be obsolete. Memantine exhibits even lower plasma concentrations of 0.4 to 1 µM after oral administration of a safe dosage of 20 mg/day, thus a 100 to 200 times higher serum concentration would be required to achieve therapeutically effective doses for reducing the virus replication.

The infection of human airways by SARS-CoV-2 does not occur homogeneously. The susceptibility of the airway epithelium for SARS-CoV-2 infection shows a gradient from highest in nasal epithelium to lowest in distal pulmonary epithelium (Hou et al., 2020). The authors suggest that the nasal cavity is the initial site of infection and lung infection could be mediated by aspiration of high-titer virus secretions from the nasopharyngeal region. Thus, keeping viral titers low in the nose secretion could be a useful strategy to reduce the severity of a SARS-CoV-2 infection and to reduce the likelihood of fatal outcomes. Reduction of nasal virus titer may generally be achieved by nose flushing with acid-buffered saline, hypertonic saline, antiseptic solutions such as ethanol or isopropanol or with antiviral solutions such as type 1 interferon (Higgins et al., 2020).

Without being bound to theory, amantadine inhibits viral replication in the Vero E6 cell system while the mode of action of amantadine on SARS-CoV-2 is unclear. Interference with the binding of the viral spike protein to ACE2 on target cells can be ruled out (Fink et al. in prep). Inhibition of a viroporin or an adjacent entry step is conceivable. Mode of action of amantadine against SARS-CoV-2 was studied by (Smieszek et al., 2020) who found a 50% reduced lysosomal uptake at 10µM amantadine. Coronaviruses use peptidases as entry receptors e.g. ACE2 by SARS-CoV and HCoV-NL63 or dipeptidyl peptidase 4 (DPP4) by MERS-CoV (Fehr and Perlman, 2015). The SARS-CoV-2 mode of infection is initiated by binding of the SARS-CoV-2 spike protein (S) to ACE2, host cell priming of S by the serine protease TMPRSS2 (Hoffmann et al., 2020).

Amantadine exerts several pharmacodynamic effects (Danysz, 2020) from which inhibition of the influenza A M2 ion channel is relevant for its antiviral efficacy against influenza A (Hay et al., 1985) Fleming 2001 (Fleming, 2001). Amantadine is also used for the treatment of Parkinson's Disease due to its low-potent non-competitive inhibition of the NMDA receptor ion channel on striatal dopaminergic neurons (Parsons et al., 1995). The viral M2 protein is considered a viroporin that forms a cation-selective ion channel which may be involved in influenza A virus uptake into the host cell (Cady et al., 2009) (Pielak and Chou, 2010). Although there is no sequence homology of the influenza A and the SARS-CoV virus it has been hypothesized that amantadine might inhibit a transmembrane channel formed by the SARS-CoV E protein (Wilson et al., 2004) (Brison et al., 2014). Substantial research on SARS-CoV was performed after the SARS-CoV pandemic in 2003. Many findings may be transferred to SARS-CoV-2. Both viruses share 77% sequence and the E protein shares even 94% of the sequence. The corona virus E proteins form a pentameric ion channel (Wilson et al., 2004; Surya et al., 2015; Singh Tomar and Arkin, 2020) and are involved in the induction of apoptosis, membrane permeability and inflammation (Nieto-Torres et al., 2015; Lalchhandama, 2020). Amantadine, which inhibits several ion channels such as the NMDA or the M2, is suspected to also inhibit Corona virus ion channels. Viroporins form proton channels that are required for the entry of the viral genome into the host cell.

Amantadine evidently inhibits the SARS-CoV-2 replication in vitro, but the required concentrations cannot be achieved by systemic administration in patients because dosages would exceed those causing toxic side effects. Topical administration allows to circumvent this problem because it allows for higher concentrations on the airway epithelium; at the same time plasma concentrations are far below the toxic symptom level.

Amantadine (1-Adamantylamine) inhalation in influenza A infected mice (22h/day, 4mg/d retained dose) was reported by Walker 1976 (Walker et al., 1976) with positive effect on survival. Wilson 1980 (Wilson et al., 1980) demonstrated in a very similar model but with higher dosing (40 mg/d retained dose) a reduced virus load. Fenton 1977 (Fenton et al., 1977) demonstrated a reduced virus load in influenza A infected ferrets that inhaled amantadine at 6mg/kg/d.

Knight 1979 (Knight et al., 1979) demonstrated in the limited number of 7 volunteers and 3 influenza patients a safe and irritation-free inhalative administration of amantadine in several protocols (1.5%, up to 9h, 2x6h, 10-11h in 3d). Hayden 1979 (Hayden et al., 1979) demonstrated a safe application of amantadine via inhalation (2x30min, 6d) in 15 healthy human volunteers; he also measured a plasma concentration of 0.13 µM amantadine after inhalative administration. The same authors (Hayden et al., 1980) demonstrated a reduced virus load and improved symptoms in 20 influenza A patients after inhalative amantadine administration (1%, 20mg/d, 3x20min, 4d). However, these initial early stage experiments neither prompted additional research regarding scope and limitations of this therapeutic approach nor motivated the practitioner to consider topical administration of amantadine to the respiratory system of the patient in their daily practice. Consequently, inhalational amantadine therapy for influenza A was never regulatorily approved.

Amantadine is commercially available from different manufacturers, including infusion solutions, capsules, tablets and syrup. Currently marketed pharmaceutical products are PK Merz infusion, Amantadin-ratiopharm and AMANTADIN-Serag which all contain the hemisulfate salt at 400 mg/l and as excipients NaCl, NaOH, HCl and water for injection.

Memantine is commercially available as hydrochloride salt in the form of tablets and as a liquid formulation in a dosing pump.

Intranasal as well as inhalative administration of amantadine have been considered in early stage research specifically only for the treatment of influenza A without providing robust and reliable results regarding safety and efficacy, but not yet for the treatment of SARS-CoV-2 airway infections.

There is a strong and ongoing demand to further improve the therapeutic options available for treating patients suffering from a SARS-CoV-2 infection. In particular, it is desirable to effectively reduce the virus replication as early as possible before significant amounts of the virus have entered the deeper areas of the respiratory system as the trachea and the bronchial tubes.

### OBJECTS OF THE INVENTION

It was an object of the present invention to improve the outcome of the treatment of patients suffering from a SARS-CoV-2 infection, in particular to provide a treatment which effectively reduces the virus replication as early as possible before significant amounts of the virus have entered the deeper areas of the respiratory system, like the trachea and the lower airways. A further object of the present invention is the provision of a method to o reduce the SARS-CoV-2 virus replication as soon as the virus is detected in the nasal cavity, with or without the patient having symptoms. A further object of the present invention is to provide a prophylactic treatment for reducing SARS-CoV-2 virus replication after exposition of the patient to proven SARS-CoV-2 positive persons.

### SUMMARY OF THE INVENTION

Surprisingly, it has been identified that the topical administration of an adamantane derivatives selected from the group of amantadine or memantine as free base or in the form of their pharmaceutically active salt to the respiratory system may advantageously be used to provide a treatment of a patient suffering from a SARS-CoV-2 infection. In particular, it was surprisingly found that the topical administration to the respiratory system of the patient allows an antiviral treatment and effective virus replication reduction at an early stage of the infection before the SARS-CoV-2 virus infects lower airways and the lung tissue resulting in pneumonia and vascular endothelitis.

Thus, in one aspect, the present invention relates to an adamantane derivative as free base or in the form of its pharmaceutically acceptable salt for use in the treatment of a patient suffering from an infection with SARS-CoV-2, wherein said adamantane derivative is selected from the group of amantadine and memantine and wherein said adamantane derivative is administered topically to the respiratory system of said patient.

In another aspect, the present invention relates to a pharmaceutical composition comprising an adamantane derivative as free base or in the form of its pharmaceutically acceptable salt for use in the treatment of a patient suffering from an infection with SARS-CoV-2, wherein said adamantane derivative is selected from the group of amantadine and memantine and wherein said adamantane derivative is administered topically to the respiratory system of said patient.

In yet another aspect, the present invention relates to a method of treating a patient suffering from an infection with SARS-CoV-2, wherein an adamantane derivative as free base or in the form of its pharmaceutically acceptable salt is administered topically to the respiratory system of said patient and wherein said adamantane derivative is selected from the group of amantadine and memantine.

### DETAILED DESCRIPTION OF THE INVENTION

The present invention may be understood more readily by reference to the following detailed description of the invention and the examples included therein.

In one aspect the invention relates to an adamantane derivative as free base or in the form of its pharmaceutically acceptable salt for use in the treatment of a patient suffering from an infection with SARS-CoV-2, wherein said adamantane derivative is selected from the group of amantadine and memantine and wherein said adamantane derivative is administered topically to the respiratory system of said patient.

In the context of the present invention, the term "adamantane derivative" refers to an organic compound having three cyclohexane rings fused to each other. Adamantane derivatives include for example a couple of pharmaceutically active compounds, including adapalene, adapromine, bromantane, carmantadine, chlodantane, dopamantine, memantine, rimantadine, saxagliptin, tromantadine and vildagliptin. The adamantane derivative of the present invention is preferably selected from the group of amantadine and memantine.

Amantadine, 1-Adamantylamine, is described, for ample, in the Merck Index, compound No. 373, Tenth Edition. A suitable way for its manufacture is described in U.S. Patent No. 3.152.180. Amantadine is a moderate affinity, uncompetitive NMDA receptor antagonist with strong voltage dependency and rapid blocking/unblocking kinetics. After therapeutic dosing major targets of amantadine based on its in vitro activity are nicotine receptors (α7, α4β2), sigma-1 receptors, AA decarboxylase, phosphodiesterase, and around its maximum therapeutic concentrations, 5-HT3 receptors and potassium channels (Danysz 2020).

Memantine, 1-amino-3,5-dimethyladamantane is an uncompetitive NMDA receptor antagonist approved for the symptomatic therapy of Alzheimer's disease (Gilling et al. 2007).

Memantine can be manufactured for example as described in US 8,138,375, US 8,436,209, US 8,796,491 and US 5,061,703.

In one preferred embodiment of the present invention, the adamantane derivative for use in the treatment of a patient suffering from an infection with SARS-CoV-2 is amantadine wherein said amantadine is administered topically to the respiratory system of said patient.

In one embodiment of the present invention, the adamantane derivative for use in the treatment of a patient suffering from an infection with SARS-CoV-2 is memantine wherein said memantine is administered topically to the respiratory system of said patient.

In another preferred embodiment of the present invention, the amantadine is administered as hydrochloride of hemisulfate salt.

In another embodiment of the present invention, the memantine is administered as hydrochloride salt.

In a particular embodiment of the present invention the adamantane derivative is administered topically by using an inhalation device selected from the group of (pressurized) metered dose inhalers (pMDIs), breath actuated inhalers, dry powder inhalers (DPI), nebulizers and soft mist inhalers. Preferably, the adamantane derivative is administered by using a nebulizer or a soft mist inhaler and wherein said nebulizer or soft mist inhaler is operated by air pressure or by ultrasound.

According to one aspect of the present invention, the adamantane derivative is administered in a daily dosage in the range of between 5 and 200 mg. The maximum total daily dosage of the adamantane derivative is in the range of between 2 and 300 mg. In another embodiment of the present invention, the adamantane derivative is administered in a daily dosage in the range of between 20 and 180 mg. In a further embodiment of the present invention, the adamantane derivative is administered in a daily dosage in the range of between 40 and 160 mg. In yet another embodiment of the present invention, the adamantane derivative is administered in a daily dosage in the range of between 60 and 140 mg. In a preferred embodiment of the present invention, the adamantane derivative is administered in a daily dosage in the range of between 160 and 200 mg. In a more preferred embodiment of the present invention, the adamantane derivative is administered in a daily dosage in the range of between 170 and 200 mg. In a furthermore preferred embodiment of the present invention, the adamantane derivative is administered in a daily dosage in the range of between 180 and 200 mg.

The adamantane derivative of the present invention is generally administered to the respiratory system of the patient in several distinct treatment sessions within 24 hours. In a preferred embodiment of the present invention, the adamantane derivative is administered to the respiratory system of the patient in several distinct treatment sessions during the daytime between 6 o'clock in the morning and 6 o'clock in the evening. In a preferred embodiment of the present invention, the adamantane derivative is administered to the respiratory system of the patient in 1, 2, 3, 4, 5 or 6 distinct treatment sessions during the daytime between 6 o'clock in the morning and 6 o'clock in the evening. In a more preferred embodiment of the present invention, the adamantane derivative is administered to the respiratory system of the patient in 6 distinct treatment sessions during the daytime between 6 o'clock in the morning and 6 o'clock in the evening.

In a preferred embodiment of the present invention, amantadine is administered topically to the respiratory system of a patient suffering from a SARS-CoV-2 infection in a total dose in the range of between 5 and 200 mg per day. In a preferred embodiment of the present invention, amantadine is administered in a daily dosage in the range of between 160 and 200 mg. In a more preferred embodiment of the present invention, amantadine is administered in a daily dosage in the range of between 170 and 200 mg. In a furthermore preferred embodiment of the present invention, amantadine is administered in a daily dosage in the range of between 180 and 200 mg.

In another preferred embodiment of the present invention, amantadine is administered topically to the respiratory system of a patient suffering from a SARS-CoV-2 infection for 2 - 14 consecutive days. In a further preferred embodiment of the present invention, amantadine is administered for 5 - 10 consecutive days. In a preferred embodiment of the present invention, amantadine in the form of its pharmaceutically acceptable salt is administered with a nebulizer every 2 to 4 hours for a time period of 15 to 45 minutes topically to the respiratory system of a patient suffering from a SARS-CoV-2 infection using a liquid formulation comprising amantadine in a concentration range of between 0.1 and 1 mg/mL. In a more preferred embodiment of the present invention amantadine in the form of its pharmaceutically acceptable salt is administered with a nebulizer every 2 hours for a time period of 30 minutes topically to the respiratory system of a patient suffering from a SARS-CoV-2 infection using a liquid formulation comprising amantadine in a concentration range of 0.4 mg/mL.

In a preferred embodiment of the present invention, memantine is administered topically to the respiratory system of a patient suffering from a SARS-CoV-2 infection in a total dose in the range of between 5 and 200 mg per day. In a preferred embodiment of the present invention, memantine is administered in a daily dosage in the range of between 160 and 200 mg. In a more preferred embodiment of the present invention, memantine is administered in a daily dosage in the range of between 170 and 200 mg. In a furthermore preferred embodiment of the present invention, memantine is administered in a daily dosage in the range of between 180 and 200 mg.

In another preferred embodiment of the present invention, memantine is administered topically to the respiratory system of a patient suffering from a SARS-CoV-2 infection for 2-14 consecutive days. In a further preferred embodiment of the present invention, memantine is administered for 5 - 10 consecutive days. In a preferred embodiment of the present invention memantine in the form of its pharmaceutically acceptable salt is administered with a nebulizer every 2 to 4 hours for a time period of 15 to 45 minutes topically to the respiratory system of a patient suffering from a SARS-CoV-2 infection using a liquid formulation comprising amantadine in a concentration range of between 5 and 15 mg/mL. In a more preferred embodiment of the present invention memantine in the form of its pharmaceutically acceptable salt is administered with a nebulizer every 2 hours for a time period of 30 minutes topically to the respiratory system of a patient suffering from a SARS-CoV-2 infection using a liquid formulation comprising amantadine in a concentration of 10 mg/mL.

According to one aspect of the present invention, the adamantane derivative is administered in the liquid form using a nebulizer. The adamantane derivative according to the present invention is generally dissolved in the form of their pharmaceutically acceptable salt in water for injection in the concentration range of between 0.1 and 50 mg/mL. Preferably, the adamantane derivative according to the present invention is dissolved in the form of its pharmaceutically acceptable salt in water for injection in the concentration range of between 0.1 and 35 mg/mL. In a further preferred embodiment, the adamantane derivative according to the present invention is generally dissolved in the form of its pharmaceutically acceptable salt in water for injection in the concentration range of between 0.1 and 25 mg/mL. In a further preferred embodiment, the adamantane derivative according to the present invention is generally dissolved in the form of its pharmaceutically acceptable salt in water for injection in the concentration range of between 0.1 and 10 mg/mL. In another preferred embodiment the liquid formulation of amantadine hydrochloride has an amantadine hydrochloride concentration of about 0.4 mg/mL. In another preferred embodiment of the present invention, the liquid formulation of amantadine hemisulfate has an amantadine hemisulfate concentration of about 0.4 mg/mL. In another preferred embodiment of the present invention the liquid formulation of memantine hydrochloride has a memantine hydrochloride concentration of about 10 mg/mL. In another preferred embodiment of the present invention the liquid formulation of memantine hydrochloride has a memantine hydrochloride concentration of about 5 mg/mL.

The pH value of the liquid formulation comprising the adamantane derivative of the present invention is generally in the range of between 5.5 and 7.0. In a preferred embodiment the pH value of the liquid preparation is between 6 and 6.5. It is generally under stood by the person skilled in the art, that the pH value of a liquid formulation comprising an adamantane derivate according to the present invention can be adjusted easily by using a suitable buffer system selected from the group of inorganic and organic acids and bases. Often a suitable buffer is based on a weak (organic) acid and its corresponding base. Buffer systems commonly used in the pharmaceutically field are selected from inorganic acids as hydrochloric acid, sulfuric acid, nitric acid, phosphoric acid, perchloric acid, bromic acid, or the like. An organic acid-based buffer can be selected from the group of acetic acid, methanesulfonic acid, ethanesulfonic acid, p-toluenesulfonic acid, fumaric acid, maleic acid, malonic acid, phthalic acid, succinic acid, lactic acid, citric acid, gluconic acid, tartaric acid, benzoic acid, pamoic acid, aspartic acid, glutamic acid, TRIS (tris(hydroxymethyl)aminomethane) or the like. In a preferred embodiment of the present invention, the liquid formulation comprising an adamantane derivative is buffered using HCl and NaOH.

Generally, there are no specific requirements for the liquid formulation of the adamantane derivative according to the present invention. As the liquid formulation is administered to the respiratory system, isotonicity of the preparation is generally considered to be advantageously. In a preferred embodiment of the present invention the liquid formulation comprising an adamantane derivative as free base or its pharmaceutically acceptable salts is based on a 0.9 % aqueous sodium chloride solution.

In a further preferred embodiment of the present invention the liquid formulation comprising an adamantane derivative and its pharmaceutically acceptable salt comprises amantadine and its pharmaceutically acceptable salt, HCl, NaOH, NaCl and water for injection.

The pharmaceutically acceptable salt the pharmaceutically acceptable salt of the adamantane derivative of the present invention is not particularly limited but may be any salt that increases the solubility of the adamantane derivative. Preferably the pharmaceutically acceptable salt of the adamantane derivative is an acid addition salt. The pharmaceutically acceptable salt must generally have low human toxicity and must not adversely affect the biological activity and physicochemical properties of the amantadine or memantine. Generally, free acids that may be used in preparation of the pharmaceutically acceptable salts may be divided into inorganic acids and organic acids. As the inorganic acid, hydrochloric acid, sulfuric acid, nitric acid, phosphoric acid, perchloric acid, bromic acid, or the like may be used. As the organic acid, acetic acid, methanesulfonic acid, ethanesulfonic acid, p-toluenesulfonic acid, fumaric acid, maleic acid, malonic acid, phthalic acid, succinic acid, lactic acid, citric acid, gluconic acid, tartaric acid, benzoic acid, pamoic acid, aspartic acid, glutamic acid, or the like may be used. Preferably, amantadine is used in the form of its hydrochloride or its hemisulfate salt. In a preferred embodiment, memantine is used in the form of its hydrochloride salt.

Generally, there are several methods known to the person skilled in the art, how to deliver an active agent like an adamantane derivative selected from the group of amantadine or memantine topically to the lungs and airways by using an inhalative method. Inhaler devices are generally used to treat or prevent diseases of the airways such as asthma, chronic obstructive pulmonary disease (COPD) or cystic fibrosis. By delivering medicine directly to the lungs, smaller doses of the medicine are needed and the onset of action of the active agent in the respiratory system is faster. As the active agent is administered topically to the affected area of the disease, a more targeted treatment is possible and systemic toxic side effect are avoided. Generally, the inhalation device can be selected from the group of (pressurized) metered dose inhalers (pMDIs), breath actuated inhalers, dry powder inhalers (DPI), nebulizers and soft mist inhalers.

(Pressurized) metered dose inhalers are also called pMDIs or aerosol inhalers. The medicine is located in a small canister which is inside a plastic case. By applying pressure to the inhaler, a measured dose of medicine comes through the mouthpiece. A metered-dose inhaler consists generally of three major components: the canister which is generally produced from aluminium or stainless steel by means of deep drawing; this canister contains the formulation; the metering valve, which allows a metered quantity of the formulation to be dispensed with each actuation; and an actuator (or mouthpiece) which allows the patient to operate the device and directs the aerosol into the respiratory system of the patient. The formulation itself contains the drug, a liquefied gas propellant and, in many cases, one or more stabilising excipients. The actuator contains the discharge nozzle and generally includes also a dust cap to prevent contamination. To use the inhaler the patient presses down on the top of the canister. Actuation of the device releases a single metered dose of the formulation which contains the medication either dissolved or suspended in the propellant. Breakup of the volatile propellant into droplets, followed by rapid evaporation of these droplets, results in the generation of an aerosol consisting of micrometer-sized medication particles that are inhaled by the patient. pMDIs require good technique and coordination by pressing down on the inhaler and breathing in at the same time. Because using the inhaler correctly can be difficult, spacer devices are recommended for use with pMDIs. The spacer is attached to the pMDI to make it easier to use the inhaler and deliver more medicine into the respiratory system. The main advantage of a metered dose inhaler is the uniformity of drug delivery, i.e. each activation of the inhaler delivers an exactly metered dose.

Dry powder inhalers are handheld devices that deliver medication to the lungs and airways as the patient inhales through it. The medication is formulated using a suitable diluent like e.g. lactose and is commonly held either in a capsule for manual loading or is located inside the inhaler. Once loaded or actuated, the patient puts the mouthpiece of the inhaler into their mouth and takes a sharp, deep inhalation (ensuring that the medication reaches the lower parts of the lungs), holding the breath for 5-10 seconds. Examples of dry powder inhalers include, but are not limited to: Turbohaler^{®}, Accuhaler^{®}, Handihaler^{®}, Ellipta inhaler^{®}, Breezhaler^{®}.

Nebulizers are a drug delivery device used to administer medication in the form of a mist inhaled into the lungs. Nebulizers are commonly used for the treatment of asthma, cystic fibrosis, COPD and other respiratory diseases or disorders. They generally use oxygen, compressed air or ultrasonic power to agitate solutions and suspensions into small aerosol droplets that are finally inhaled from the mouthpiece of the device. The different systems include for example jet nebulizers, soft mist inhalers, ultrasonic wave inhalers, vibrating mesh inhalers. The most commonly used nebulizers are jet nebulizers. Jet nebulizers are also commonly known as "atomizers". Jet nebulizers are connected by tubing to a supply of compressed gas, usually compressed air or oxygen to flow at high velocity through a liquid pharmaceutical preparation to turn it into an aerosol that is inhaled by the patient. The Soft Mist Inhaler Respimat^{®} was introduced by Boehringer Ingelheim in 1997. This technology provides a metered dose to the patient, when the bottom of the inhaler is rotated 180 degrees by hand, transferring the tension into a spring around the flexible liquid container. When the user activates the bottom of the inhaler, the energy from the spring is released and imposes pressure on the flexible liquid container, causing the liquid to spray out of nozzles, thus forming a soft mist to be inhaled. The device features no gas propellant and no need for battery/power to operate. The average droplet size in the mist was measured to 5.8 micrometers. Ultrasonic wave nebulizers are in use since 1965. The technology is based on an electronic oscillator to generate a high frequency ultrasonic wave, which causes the mechanical vibration of a piezoelectric element. This vibrating element is in contact with a liquid reservoir and its high frequency vibration is sufficient to produce a vapor mist. The ultrasonic Vibrating Mesh Technology (VMT) represents a further improvement of an ultrasonic wave nebulizer. With this technology a mesh/membrane with 1000-7000 laser drilled holes vibrates at the top of the liquid reservoir, and thereby pressures out a mist of very fine droplets through the holes. vibrating mesh nebulizers. Available VMT nebulizers include, for example: Pari eFlow, Respironics i-Neb, Beurer Nebulizer IH50, and Aerogen Aeroneb. Nebulizers uses generally their medicine in the form of a liquid solution, which is often loaded into the device directly upon use. Usually, the aerosolized medicine is inhaled through a tube-like mouthpiece, similar to the system used with an inhaler. The mouthpiece can be sometimes replaced with a face mask, as it is used for inhaled anesthesia. Masks are in particular advantageous for ease of use with young children or the elderly.

In a preferred embodiment of the present invention the adamantane derivative for use is administered by using a nebulizer. In particular, nebulizers using compressed air or ultrasound can be advantageously used for administering the adamantane derivative of the present invention. In an alternative embodiment, breath-operated dry powder inhalers can be used for administering the adamantane derivative according to the present invention.

In the context of the present invention, the terms inhalative treatment means an administration of the active agent to the intranasal area, the mouth and/or the respiratory system. In a preferred embodiment of the present invention, amantadine or memantine and their pharmaceutically acceptable salts are administered to the nasal cavity, the nasopharynx, the tracheal, the bronchial and alveolar surface.

In a further embodiment of the present invention the adamantane derivative is administered to a patient suffering from a SARS-CoV-2 infection using a continuous or intermittent aerosol inhalation. The continuous or intermittent administration of the adamantane derivative of the present invention can be performed by using a face mask or an intranasal tube. In another embodiment of the present invention the adamantane derivative can be administered to a patient suffering from a SARS-CoV-2 infection by using an intranasal spray application or an intranasal liquid instillation in intervals of 1 to 6 hours. In a preferred embodiment of the present invention, the adamantane derivative can be administered to a patient suffering from a SARS-CoV-2 infection by using an intranasal spray application or an intranasal liquid instillation in intervals of 2 to 4 hours. In a more preferred embodiment of the present invention, the adamantane derivative can be administered to a patient suffering from a SARS-CoV-2 infection by using an intranasal spray application or an intranasal liquid instillation in intervals of 2 hours.

In a preferred embodiment of the present invention, the intranasal or inhalative administration of the adamantane derivative to the patient suffering from a SARS-CoV-2 infection can be performed by using a regulatory approved and commercially available infusion solution, such as PK Merz (amantadine hemisulfate in an 1mM aqueous solution). In cases higher concentrations are needed, the preparation can be manufactured by the hospital pharmacy. Amantadine hydrochloride solutions as well as hemisulfate solutions are also available as generic drugs in many countries.

In a further embodiment, the adamantane derivatives of the present invention can be administered together with at least one standard agent to treat a patient suffering from an infection with SARS-CoV-2 selected from remdesivir, fluticasone, flunisolide, beclometasone, budesonide or combinations thereof.

In another aspect, the present invention relates to a pharmaceutical composition comprising an adamantane derivative as free base or in the form of its pharmaceutically acceptable salt for use in the treatment of a patient suffering from an infection with SARS-CoV-2 wherein said adamantane derivative is selected from the group of amantadine and memantine and wherein said adamantane derivative is administered topically to the respiratory system of said patient. For preparing a pharmaceutical preparation comprising the adamantane derivative of the present invention, the adamantane derivative can be formulated by various techniques dependent on the desired application purposes which are known in the art. For example, the adamantane derivative can be used in combination with one or more pharmaceutically acceptable carriers as a pharmaceutical composition. The pharmaceutically acceptable carrier(s) must be acceptable in the sense of being compatible with the other ingredients of the formulation and being not deleterious to the recipient thereof. The pharmaceutical carrier employed may include a solid, a gel, or a liquid. Exemplary of solid carriers are lactose, terra alba, sucrose, talc, gelatine, agar, pectin, acacia, magnesium stearate, stearic acid and the like. Exemplary of liquid carriers are glycerol, phosphate buffered saline solution, water, emulsions, various types of wetting agents, and the like. Suitable carriers comprise those mentioned above, and others well known in the art, see, e.g., Remington's Pharmaceutical Sciences, Mack Publishing Company, Easton, Pennsylvania. In a preferred embodiment of the present invention the pharmaceutical carrier for the adamantane derivative is a liquid. In an aspect, the pharmaceutical composition can be dissolved in a diluent, prior to administration. The diluent is also selected so as not to affect the biological activity of the adamantane derivative. Examples of such diluents are distilled water or physiological saline. In addition, the pharmaceutical composition or formulation may also include other carriers or non-toxic, non-therapeutic, non-immunogenic stabilizers and the like. Thus, the formulated adamantane derivative can be present, in an aspect, in liquid or lyophilized form. The formulated adamantane derivative may be used for human therapy of a patient suffering from a SARS-CoV-2 infection in a therapeutically effective dose.

In the context of the present invention, the term "comprises" or "comprising" means "including, but not limited to". The term is intended to be open-ended, to specify the presence of any stated features, elements, integers, steps or components, but not to preclude the presence or addition of one or more other features, elements, integers, steps, components, or groups thereof. The term "comprising" thus includes the more restrictive terms "consisting of" and "consisting essentially of".

In the context of the present invention, the term "treating a patient suffering from a SARS-CoV-2 infection" means that the treatment reduces the viral load in the treated area of the patient. In particular, the treatment is intended to avoid severe and fatal developments of the infection.

In particular embodiments, the pharmaceutical composition comprising an adamantane derivative according to the present invention is for use in treating a patient suffering from a SARS-CoV-2 infection wherein said adamantane derivative is selected from the group of amantadine and memantine and wherein said adamantane derivative is administered topically to the respiratory system of said patient.

In another aspect, the present invention relates to a method of treating a of treating a patient suffering from an infection with SARS-CoV-2 wherein the method comprises the administration of a therapeutically effective amount of an adamantane derivative wherein said adamantane derivative is selected from the group of amantadine and memantine and wherein said adamantane derivative is administered topically to the respiratory system of said patient.

### EXAMPLES

### Example 1: In-vitro activity in Vero E6* cells

Cytotoxicity and antiviral activity of amantadine hydrochloride was studied in Vero E6 cells (Cercopithecus aethiops, kidney, ATCC CRL-1586). The cell line is routinely maintained in DMEM supplemented with 1% glutamine, 1% penicillin/streptomycin and 10% fetal bovine serum and can be infected with SARS-CoV-2 (Ogando et al., 2020).

### Antiviral activity assay with nucleoprotein readout (3rd experiment)

Exponentially growing Vero E6 cells were seeded into a 96-well plate at their optimal density in complete medium. 24 hours later cells were infected with SARS-CoV-2 (viral strain INMI1) at 0.01 moi (multiplicity of infection) and then exposed to different concentrations of the drug (0-0.1-1-10-100-300 µM for amantadine hydrochloride) for 72 hours. Drug dilutions were performed in culture medium. Replicates for each concentration point were examined. At the end of the incubation period, antiviral activity was examined through both ELISA (Sino Biological, quantifying SARS-CoV-2 nucleoprotein) as well as a cytoprotection assay (toxicity effect examined through an inverted microscope). An inhibitory concentration 50% (IC50) value was calculated through interpolation of the dose-response curves generated.

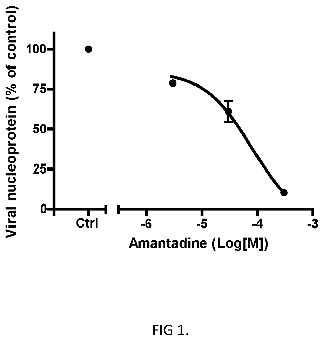

**Figure 1** Effect of amantadine hydrochloride on SARS-CoV-2 replication in Vero E2 cells (IC50 = 88 µM) in the third series of experiments. The y-axis shows the virus nucleoprotein in % of that in the supernatant of an infected but untreated control cell culture. The nucleoprotein was quantified by ELISA. Results are means ± SD of 2-4 experiments.

### Example 2: Treatment patients suffering from SARS-CoV-2 using amantadine hemisulfate administered using a nebulizer.

A 50 year old male patient who had contact 2 days ago with a colleague who turned out to be SARS-CoV-2 positive the day after the meeting, consults his primary care physician. He reports a sore throat but does not have elevated temperature. The RT-PCR test for SARS-CoV-2 of a nasal swab is also positive; HR is 78/min, BP is 120/80mmHg, BR is 15/min, peripheral O2 saturation is 97%, no further health issues are diagnosed. The patient is diagnosed with Covid-19 and isolated in home quarantine with daily doctor calls to evaluate his health status. He is prescribed an amantadine hemisulfate solution with 0.4mg/ml to inhale every 2 h for 30min 6 times per day with a home ultrasound inhaler with face mask. The treatment is continued for 7 days. The patient develops transiently minor fever and does not suffer from breathing problems. He recovers within 14 days after treatment start.

## Claims

1. An adamantane derivative as free base or in the form of its pharmaceutically acceptable salt for use in the treatment of a patient suffering from an infection with SARS-CoV-2 wherein said adamantane derivative is selected from the group of amantadine and memantine and wherein said adamantane derivative is administered topically to the respiratory system of said patient.

2. An adamantane derivative for use according to claim 1, wherein said adamantane derivative is administered by using an inhalation device selected from the group of pressurized metered dose inhalers (pMDIs), breath actuated inhalers, dry powder inhalers (DPI), nebulizers and soft mist inhalers.

3. An adamantane derivative for use according to claim 2, wherein said adamantane derivative is administered by using a nebulizer or a soft mist inhaler and wherein said nebulizer or soft mist inhaler is operated by air pressure or by ultrasound.

4. An adamantane derivative for use according to claim 1 or 2, wherein the adamantane derivative is amantadine.

5. An adamantane derivative for use according to claim 4, wherein the amantadine is administered in a total dose in the range of between 5 and 200 mg per day.

6. An adamantane derivative for use according to claim 4, wherein the amantadine is administered for 2 - 14 consecutive days.

7. An adamantane derivative for use according to claim 4, wherein the amantadine is administered as hydrochloride or hemisulfate salt.

8. An adamantane derivative for use according to claim 1 or 2, wherein the adamantane derivative is memantine.

9. An adamantane derivative for use according to claim 8, wherein the memantine is administered in a total dose in the range of between 5 and 200 mg per day.

10. An adamantane derivative for use according to claim 8, wherein the memantine is administered for 2-14 consecutive days.

11. An adamantane derivative for use according to claim 8, wherein the memantine is administered as hydrochloride salt.

12. An adamantane derivative for use according to claim 1 to 11, wherein said adamantane derivative is administered together with at least one standard agent to treat a patient suffering from an infection with SARS-CoV-2 selected from remdesivir, fluticasone, flunisolide, beclometasone, budesonide or combinations thereof.

13. A pharmaceutical composition comprising an adamantane derivative for use in treating a patient suffering from an infection with SARS-CoV-2 according to any one of claims 1 to 12.

14. A method of treating a patient suffering from an infection with SARS-CoV-2 wherein the method comprises the administration of a therapeutically effective amount of an adamantane derivative according to any one of claims 1 to 13.
